Europäisches Patentamt

European Patent Office

Office européen des brevets

(19)

(11) Veröffentlichungsnummer: **0 355 570**
**A2**

(12) **EUROPÄISCHE PATENTANMELDUNG**

(21) Anmeldenummer: 89114628.4

(22) Anmeldetag: 08.08.89

(51) Int. Cl.4: **C12N 9/00 , C12N 9/24 , A23L 3/346 , A61K 37/48 , A61K 37/62 , A01N 63/02 , //C12N1/06**

(30) Priorität: 12.08.88 DE 3827391
20.08.88 DE 3828306

(43) Veröffentlichungstag der Anmeldung:
28.02.90 Patentblatt 90/09

(84) Benannte Vertragsstaaten:
**BE CH DE FR GB IT LI NL**

(71) Anmelder: HOECHST AKTIENGESELLSCHAFT
Postfach 80 03 20
D-6230 Frankfurt am Main 80(DE)

(72) Erfinder: **Then, Johann, Dr.**
**Bechtenwaldstrasse 70**
**D-6230 Frankfurt am Main 80(DE)**
Erfinder: **Lotz, Andreas, Dr.**
**Parkstrasse 10**
**D-6233 Kelkheim (Taunus)(DE)**
Erfinder: **Klug, Christian, Dr.**
**Friedrich-Ebert-Strasse 15**
**D-6231 Schwalbach am Taunus(DE)**
Erfinder: **Wöhner, Gerhard, Dr.**
**Flörsheimer Strasse 27**
**D-6093 Flörsheim am Main(DE)**

(54) Zubereitung zur Konservierung mikrobiell verderblicher Produkte.

(57) Eine Zubereitung bestehend aus einem Pilz-lysierenden Enzymprodukt eines Streptomyceten und β-1,3-Glucanase eignet sich hervorragend zur Konservierung von mikrobiell verderblichen Produkten.

EP 0 355 570 A2

## Zubereitung zur Konservierung mikrobiell verderblicher Produkte

Die mikrobielle Produktion von Metaboliten, mit deren Hilfe das Wachstum von Pilzen unterdrückt werden kann, wurde verschiedentlich beschrieben. In dem US-Patent 4 534 965 wird ein Mittel offenbart, mit dessen Hilfe phytopathogene Pilze abgetötet werden können. Dieses Mittel wird durch Kultivierung des Streptomyces-Stamms ATCC 39434 auf einem Chitin-haltigen Substrat gewonnen.

In der Europäischen Patentanmeldung 0171381 werden Mikroorganismen beschrieben, mit deren Hilfe das Wachstum von Nematoden unterdrückt werden kann. Es werden Pseudomonaden mit fremden Genen, die für Chitinase und Glycosidase kodieren, transformiert, um durch höhere Enzymausschüttung eine stärkere wachstumsunterdrückende Wirkung auf die Nematoden ausüben zu können.

Ebenfalls ein mit einem Chitinase-Gen transformiertes Bakterium wird in der Europäischen Patentanmeldung 0157351 erwähnt.

Die nach den in den genannten Dokumenten beschriebenen Methoden hergestellte Chitinase kann zur Konservierung von Saatgut verwendet werden.

In der Deutschen Patentanmeldung P 38 27 392.6 wurde vorgeschlagen, ein Pilz-lysierendes Enzymprodukt durch Kultivierung von Streptomyces spec. DSM 4666 herzustellen. Der Stamm wurde am 10.6.1988 bei der Deutschen Sammlung von Mikroorganismen und Zellkulturen GmbH nach dem Budapester Vertrag unter obengenannter Nummer hinterlegt. Überraschend wurde nun ein Mittel, bestehend aus dem Pilzlysierenden Enzymprodukt der Deutschen Patentanmeldung und $\beta$-1,3-Glucanase, mit dessen Hilfe die Haltbarkeit mikrobiell verderblicher Produkte wesentlich verlängert werden kann, gefunden.

Die Erfindung betrifft somit:

1. Eine Zubereitung enthaltend das Pilz-lysierende Enzymprodukt, erhältlich durch Kultivierung von Streptomyces spec. DSM 4666, und $\beta$-1,3-Glucanase in einem Gewichtsverhältnis von 1:100 bis 100:1.

2. Die unter 1. charakterisierte Zubereitung zur Anwendung als Konservierungsmittel.

3. Ein Verfahren zur Herstellung der unter 1. charakterisierten Zubereitung, das dadurch gekennzeichnet ist, daß das Pilz-lysierende Enzymprodukt und die $\beta$-1,3-Glucanase in eine geeignete Applikationsform gebracht werden.

Im folgenden wird die Erfindung, insbesondere in ihren bevorzugten Ausführungsformen detailliert beschrieben. Ferner ist die Erfindung in den Ansprüchen definiert.

Die Herstellung des Pilz-lysierenden Enzymprodukts ist ausführlich in der Deutschen Patentanmeldung P 38 27 392.6 beschrieben. Es kann durch Kultivierung von Streptomyces spec. DSM 4666 in einem Chitin und eine Stickstoffquelle sowie die üblichen Nährstoffe enthaltenden Nährmedium gewonnen werden.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40°C, vorzugsweise bei etwa 25 bis 30°C, insbesondere bei 28 bis 30°C erfolgen. Man kultiviert den Mikroorganismus unter den genannten Bedingungen bis die stationäre Phase erreicht ist, etwa 60 bis 120 Stunden, bevorzugt 60 bis 75 Stunden.

Das Pilz-lysierende Enzymprodukt kann wie folgt charakterisiert werden:
- Temperaturstabilität bis zu 70°C,
- Temperaturoptimum 40 bis 60°C, insbesondere 45 bis 55°C,
- pH-Stabilität von pH 4 bis 10, insbesondere von 5 bis 7,
- pH-Optimum im Bereich von 4,5 bis 6, insbesondere 5 bis 5,5.

Die $\beta$-1,3-Glucanase kann aus pflanzlichen oder mikrobiellen Quellen gewonnen werden. Das Enzym ist beispielsweise als Laminarinase von der Firma Sigma, Taufkirchen, BRD zu beziehen.

Unter mikrobiell verderblichen Produkten, werden insbesondere solche verstanden, die einem Verderb durch Pilzbefall ausgesetzt sind. Hierzu gehören Lebensmittel, wie beispielsweise Backwaren, Obstprodukte oder Fleisch- und Wurstwaren, Käse und Milchprodukte, Feinkostartikel, Fertigprodukte sowie Getränke, pharmazeutische Produkte, wie Salben, kosmetische Produkte, Futtermittel einschließlich Petfood, Tabak, Tabakerzeugnisse und Verpackungsmaterialien.

Die Wirksubstanzen können gleichzeitig oder aufeinanderfolgend auf das Produkt aufgebracht werden. Je nach Anwendungszweck werden die Bestandteile der Zubereitung in einem Gewichtsverhältnis von 1 zu 100 bis 100 zu 1, vorzugsweise 1 zu 50 bis 50 zu 1, gemischt und dem zu konservierenden Produkt nach herkömmlichen Verfahren zugesetzt. Die Zugabe der Mischung erfolgt in einer Größenordnung, daß 0,1 bis 200 U, vorzugsweise 2 bis 100 U, des Pilz-lysierenden Enzymprodukts und 0,1 bis 100 U der $\beta$-1,3-Glucanase, bevorzugt 1 bis 50 U, vorzugsweise 2 bis 10 U, pro g des zu konservierenden Produkts in diesem enthalten sind. 1 U bezieht sich auf die Enzymmenge, die 1 $\mu$mol reduzierende Kohlenhydrate pro Minute aus Chitin bzw. Laminarin freisetzt.

Das Mittel ist in einem pH-Bereich zwischen 2 bis 7, vorzugsweise 4,5 bis 6 wirksam.

Der Vorteil der Mischung besteht darin, daß man ganz auf die Verwendung chemischer Konservierungsmittel in durch Pilz-befall gefährdeten Produkte verzichten kann, wodurch eine geschmackliche Verbesserung erreicht werden kann.

Selbstverständlich kann oder sollte die erfindungsgemäße Wirkstoffkombination nur dann sinnvoll eingesetzt werden, wenn die zu konservierenden Produkte unter hygienisch einwandfreien Bedingungen hergestellt worden sind und eine niedrige Anfangskeimzahl aufweisen.

In den folgenden Beispielen wird die Erfindung weitergehend erläutert. Die Prozentangaben beziehen sich auf das Gewicht, wenn nicht anders angegeben.

**Beispiel 1**

Lysewirkung auf lebende Pilze

a) Ein Schrägagarmedium bestehend aus

| Malzextrakt | 20 g/l |
| Hefeextrakt | 2 g/l |
| Glucose | 10 g/l |
| $(NH_4)_2HPO_4$ | 0,5 g/l |
| Agar | 15 g/l |

wurde mit Aspergillus niger (DSM 1957) beimpft. Nach 5 Tagen Wachstum bei 25° C wurden die Sporen mit 5 ml 0,9 % NaCl-Lösung abgeschwemmt. Mit 50 µl dieser Sporensuspension wurden 5 ml folgender Nährlösung beimpft:

| Glucose | 10 g/l |
| Caseinpepton | 4 g/l |
| Fleischextrakt | 4 g/l |
| Hefeextrakt | 0,5 g/l |
| Leberextrakt | 0,5 g/l |
| NaCl | 2,5 g/l. |

Der pH wurde mit HCl auf 5,0 eingestellt und die Kultur mit 150 UPM bei 25° C geschüttelt. Nach ca. 48 h treten bei Wachstum des Stammes dicke Myzelbrocken auf, so daß die optische Dichte nicht mehr meßbar war. Die wachstumshemmende Wirkung des erfindungsgemäßen Gemischs, bestehend aus Pilz-lysierendem Enzymprodukt aus Streptomyces spec. DSM 4666 (EP) und β-1,3-Glucanase, auf die Kultur wurde photometrisch anhand der optischen Dichte bei 650 nm bestimmt. Die Zusammenfassung der Ergebnisse ist in Tabelle 1 aufgeführt.

Tabelle 1

| Medium | optische Dichte | | |
| --- | --- | --- | --- |
| | 24 h | 28 h | 48 h |
| Kulturlösung (KL) | 0,50 | 0,82 | Myzel |
| KL + β-1,3-Glucanase 8 U/ml | 0,32 | 1,02 | Myzel |
| KL + β-1,3-Glucanase 20 U/ml | 0,27 | 1,2 | Myzel |
| KL + β-1,3-Glucanase 8 U/ml + EP 80 U/ml | 0,03 | 0,04 | 0,03 |
| KL + β-1,3-Glucanase 8 U/ml + hitze inaktiviertes EP | 0,45 | 0,98 | Myzel |

Das erfindungsgemäße Enzymprodukt (EP) wurde gemäß Beispiel 3 der Deutschen Patentanmeldung P 38 27 392.6 gewonnen.

Die Aktivitätsmessung des Pilz-lysierenden Enzymprodukts erfolgte nach folgender Methode: zu 1,5 ml Chitinlösung (Sigma-Chitin Nr. C 3641; 4 mg Chitin/ml Aqua bidest.) wurden 0,38 ml Kulturüberstand gegeben. Der gesamte Ansatz wurde 1 h bei 45°C geschüttelt, so daß das Chitin fein suspendiert blieb. Dann wurde zentrifugiert und 0,5 ml aus dem Überstand mit 1 ml Dinitrosalicylsäurereagenz gemischt. Das Reagenz besteht aus 10 g 3,5-Dinitrosalicylsäure, 300 g K-Na-Tartrat, 16 g NaOH in 1 l Wasser. Die Lösung ist lichtempfindlich.

Die Mischung wurde 5 min. in siedendem Wasser erhitzt, abgekühlt, 1:10 mit Wasser verdünnt und im Photometer bei 530 nm gegen einen Leerwert gemessen. Der Leerwert bestand aus 0,38 ml hitzeinaktiviertem Kulturüberstand (5 min. in siedendem Wasser), der mit 1,5 ml Chitinlösung gemischt wurde und dann wie eine normale Probe behandelt wurde.

Die Aktivitätsmessung der $\beta$-1,3-Glucanase erfolgt analog mit Laminarin aus Laminaria digitata (Sigma Nr. L 9634) als Substrat.

b) Wie in Beispiel 1 wurden 5 ml Nährlösung mit 50 $\mu$l einer Sporensuspension von Fusarium oxysporium DSM 2018 beimpft und 24 h wachsen gelassen. Dann erfolgte die Zugabe von $\beta$-1,3-Glucanase 8 U/ml und EP (nach Beispiel 3 der Deutschen Patentanmeldung P 38 27 392.6) 80 U/ml. Tabelle 3 ergibt den weiteren Wachstumsverlauf:

Tabelle 3

| | 0 h | 24 h | 28 h | 48 h |
|---|---|---|---|---|
| Kulturlösung | 0,10 | 0,856 | 1,29 | 1,80 |
| Kulturlösung + Enzymmischung (Zugabe nach 24 h) | 0,12 | 0,62 | 0,56 | 0,19 |

**Beispiel 2**

Lysewirkung auf Pilzsporen

Wie in Beispiel 1 beschrieben, wurden Sporensuspensionen hergestellt. Die Wirkung von Enzymmischungen auf das Wachstum von Pilzsporen bzw. Hefen wurde geprüft und in Tabelle 2 zusammengefaßt:

a = nur Kulturlösung

b = Kulturlösung mit $\beta$-1,3-Glucanase 8 U/ml und EP (nach Beispiel 3 der Deutschen Patentanmeldung 38 27 392.6) 80 U/ml.

Tabelle 2

| Stamm | optische Dichte 48 h |
|---|---|
| Fusarium oxysporium | |
| DSM 2018 | |
| a<br>b | 1,1<br>0,03 |
| Geotrichum candidum | |
| CBS 18767 | |
| a<br>b | Myzel<br>0,05 |
| Byssochlamus nivea | |
| CBS 60871 | |
| a<br>b | Myzel<br>0,04 |
| Saccharomyces cerevisae | |
| DSM 1333 | |
| a<br>b | 1,56<br>1,34 |
| Candida lipolytica | |
| ATCC 20383 | |
| a<br>b | 1,47<br>0,30 |
| Schizosaccharomyces pombe | |
| DSM 70577 | |
| a<br>b | 1,19<br>0,45 |
| Debaryomyces hansenii | |
| DSM 70238 | |
| a<br>b | 2,23<br>0,10 |
| Penicillium roqueforti | |
| DSM 1079 | |
| a<br>b | Myzel<br>0,01 |

**Beispiel 3**

Konservierung von Apfelsaft

Apfelsaft wurde sterilfiltriert und in sterilen Kolben mit je 50 µl einer Sporenabschwemmung von
Fusarium oxysporium DSM 2018
Penicillium patulum DSM 62862
Byssochlamus nivea CBS 60871
beimpft. Die Säfte wurden mit den entsprechenden Zusätzen (s. Tabelle 4) versehen und bei 30°C und
Raumtemperatur gelagert. Das Wachstum der Pilze wurde durch makroskopische Beobachtung und
Bestimmung der optischen Dichte bei 650 nm verfolgt.

a = Apfelsaft

b = Apfelsaft + $\beta$-1,3-Glucanase 8 U/ml + EP (nach Beispiel 3 der Deutschen Patentanmeldung P 38 27
392.6) 80 U/ml.

Tabelle 4

| Stamm | Zeit (Tage) | | |
|---|---|---|---|
| | 0 | 6 | 11 |
| Fusarium oxysporium | | | |
| a<br>b | 0,1<br>0,3 | 1,4<br>0,6 | 1,9<br>0,39 |
| Penicillium patulum | | | |
| a<br>b | 0,1<br>0,3 | 0,9<br>0,55 | Myzel<br>0,32 |
| Byssochlamus nivea | | | |
| a<br>b | 0,1<br>0,35 | Myzel<br>0,65 | Myzel<br>0,58 |

## Ansprüche

1. Zubereitung enthaltend das Pilz-lysierende Enzymprodukt, erhältlich durch Kultivierung von Streptomyces spec. DSM 4666, und $\beta$-1,3-Glucanase in einem Gewichtsverhältnis von 1 zu 100 bis 100 zu 1.

2. Zubereitung nach Anspruch 1, dadurch gekennzeichnet, daß die Zusammensetzung einem Gewichtsverhältnis von 50:1 bis 1:50 entspricht.

3. Verwendung der Zubereitung nach Anspruch 1 oder 2 als Konservierungsmittel.

4. Verwendung nach Anspruch 3, in Lebensmitteln, kosmetischen und pharmazeutischen Produkte, Saatgut, Verpackungsmaterialien, Tabak und Tabakerzeugnissen sowie Futtermitteln.

5. Verwendung nach Anspruch 3 oder 4, dadurch gekennzeichnet, daß dem zu konservierenden Produkt die Zubereitung in einer Menge zugesetzt wird, daß 0,1 bis 200 U des pilzlysierenden Enzymprodukts sowie 0,1 bis 100 U der $\beta$-1,3-Glucanase pro g darin enthalten sind.

6. Verwendung nach Anspruch 5, dadurch gekennzeichnet, daß 2 bis 100 U des pilzlysierenden Enzymprodukts sowie 1 bis 50 U der $\beta$-1,3-Glucanase enthalten sind.

7. Verfahren zur Herstellung der Zubereitung nach Anspruch 1 oder 2, dadurch gekennzeichnet, daß das Pilz-lysierende Enzymprodukt und $\beta$-1,3-Glucanase in eine geeignete Applikationsform gebracht werden.